# EUROPEAN PATENT SPECIFICATION

(11) **EP 0 710 470 B1**
(45) Date of publication and mention of the grant of the patent: **22.03.2000**
(21) Application number: 94119091.0
(22) Date of filing: 03.12.1994
(51) Int. Cl.: A61F 13/15

(54) **High speed mechanical straining process to provide extensibility to laminates for use in disposable absorbent articles**
Verfahren zum Strecken, mit hoher Geschwindigkeit, zur Verbesserung der Dehnbarkeit von Laminaten zur Anwendung in absorbierenden Wegwerfprodukten
Procédé d'étirage, à grande vitesse, pour améliorer l'extensibilité des laminés utilisés pour des articles absorbant à jeter

(30) Priority: 20.10.1994 EP 94203040
(43) Date of publication of application: 08.05.1996
(73) Proprietor: THE PROCTER & GAMBLE COMPANY, Cincinnati, Ohio 45202 (US)
(72) Inventor: Geilich, Ralf, D-74564 Crailsheim-Rossfeld (DE); Plumley, Julian Ashton, D-74589 Satteldorf (DE)
(74) Representative: Hirsch, Uwe Thomas

(56) References cited:
- WO-A-93/11725
- US-A- 4 146 586
- US-A- 4 940 462
- US-A- 5 064 492
- US-A- 5 354 400

## Description

### FIELD OF THE INVENTION

The present invention relates to a process for making disposable absorbent articles such as sanitary napkins, panty liners, and incontinence pads that have side wrapping elements for folding around the side edges of the wearer's undergarment on each lateral side of the article. The components are designed to automatically wrap the sides of a wearer's undergarments when the undergarments are pulled up by having zones of extensibility. Importantly the zones of extensibility are created by a high speed straining process of a laminate which comprises two layers at least one of which has apertures. The layers at least in the extensible zones are joined to each other by a soldering step.

### Background of the invention

Absorbent articles such as sanitary napkins, pantiliners, and incontinence pads are devices that are typically worn in the crotch region of an undergarment. These devices are designed to absorb and retain liquid and other discharges from the human body and to prevent body and clothing soiling. Sanitary napkins are a type of absorbent article worn by women in a pair of panties that is normally positioned between the wearer's legs, adjacent to the perennial area of the body. Sanitary napkins in particular with side wrapping elements, often also referred to as side flaps or wings, are disclosed in the literature and are available in the marketplace.

Sanitary napkins having wings or side flaps of various types are disclosed in U.S. Patent 4,687,478, U.S. Patent 4,608,047, U.S. Patent 4,589,876, Reexamination Patent No. B1 4,589,876, U.S. Patent 4,285,343. Sanitary napkins having wings are commonly viewed as providing good protection against soiling.

However, some women find applying sanitary napkins having side flaps to be inconvenient for various reasons. For instance, some women find it to be difficult to attach the side flaps to the underside of the crotch of their panties. This can be due to factors such as difficulties in folding the side flaps property into place and to stick them to the sanitary napkin. As a result, some women still prefer a sanitary napkin without side flaps. In addition, some women who generally prefer a sanitary napkin with side flaps, occasionally (such as during periods of light flow) prefer a sanitary napkin without. Therefore, there is a need for a sanitary napkin which provides an alternative to sanitary napkins having conventional side flaps while still providing a similar protection.

Several variations of sanitary napkins having conventional side flaps have been suggested. For example, U.S. Patent 4,911,701 discloses a sanitary napkin having elastic strands for providing a greater convex shape to the body-facing portion of the central absorbent and for enabling adhesive-free placement of the side flaps into the panties. U.S. Patent 4,940,462 discloses a sanitary napkin with longitudinally expandable flaps. The flaps are designed to fold over the exterior of the wearer's panty and then to expand to conform with the contour of the panties. Further improvements of side wrapping elements with extensible zones have been disclosed in WO 95/0767; and WO 95/0302 and EP-A-0 695 542. US-A-5,354,400 discloses sanitary napkins comprising wings or side flaps which are rendered laterally extensible by ring-rolling or corrugating. These flaps can be formed from laminates.

In these disclosures the extensibility can be provided by a number of different processes. For example the extensible zones can be created by mechanically straining, corrugating, "ring-rolling", heating and deforming, subjecting portions of the side wrapping elements or flops to compression between mating plates, and the like.

In particular high speed mechanical straining such as corrugating or ring-rolling are desirable due to the manufacturing efficiency from allowing high speed production. Also ring-rolled zones of extensibility can have an angled extensibility relative to the machine direction (process transport direction). Suitable methods for ring-rolling are described in U.S. Patent 4,107,364, U.S. Patent 4,834,741, U.S. Patent 5,143,679, U.S Patent 5,156,793 and U.S. Patent 5,167,897.

Particularly preferred side wrapping elements or flaps comprise at least two layers of material but sometimes more, for example four layers of materials which are joined together forming a laminate that is rendered extensible only after being formed into a laminate. In particular prior art sanitary napkins with side wrapping elements having extensible zones are constructed such that the side wrapping elements are formed by laterally extending parts of the uppermost layer of the sanitary napkin (typically called topsheet) and the lowermost layer of the sanitary napkin (typically designated the backsheet).

The laminate formed in the area laterally outside the main portion of the sanitary napkin is strained by mechanical processes like the aforementioned ring-rolling at the production speed of these articles. For the laminate to display uniform behaviour during the mechanical straining the individual layers need to be properly joined to each other at least in the areas of mechanical straining.

Typically adhesives, especially hot melt adhesives have been suggested and used for that purpose. A large variety of adhesives for the different situations occurring in the manufacturing process of disposable absorbent articles have been developed and continue to be developed. Alternative joining methods include welding which is used between plastic materials of similar kinds allowing to create areas where the materials are fused to each other to create permanent connections or crimping which is a local mechanical deformation of the layers such that the layers interlock locally.

It now has been found that an alternative method of joining materials, namely soldering, can beneficially be used in the process of high speed mechanical straining. This method as such is known from the art of joining metals but so far has not been applied in the field of disposable absorbent articles.

A well known problem occurring when adhesively joining laminates prior to straining is that adhesive can migrate through permeable materials. This may happen while the adhesive is applied and also thereafter. If impermeable materials are joined to each other the straining process can create small apertures through which the adhesive then migrates.

Adhesives used in the field of disposable absorbent articles are tackified to display adhesive characteristics over a broad range of temperatures. They are usually still tacky at room temperature which makes their presence on the outside of a laminate undesirable in general.

It is particularly undesirable during the straining process to have adhesive on the outside of any of the laminates because adhesive build up, in particular sticky adhesive build up on fast moving machinery parts quickly leads to unstable process conditions. As a minimum the occurrence of these conditions require frequent cleaning but can even cause disastrous material destruction leading to machine stops and reduce efficiency.

Also adhesive which may be present on the outside of disposable absorbent articles is highly undesirable for the consumer. If adhesives which are tacky at the usage temperature of these products, contact the wearer's skin or garment they will usually cause residue adhesive on garments or irritation to the wearer's skin.

These drawbacks of adhesives can be overcome by the alternative process of welding or crimping. Welding of materials usually requires material similarities if not identity in order to be feasible at all. Welding also has the disadvantage of requiring very exact temperature controls in order to provide a proper bond between materials while not causing melting away of materials or so called welding holes. This problem of very tight controls or material incompatibility to welding altogether is significantly reduced with today's adhesive technology which however carries the above mentioned drawbacks. Also welding is usually not possible for a full surface but can only be done along lines or in small areas.

The other alternative to adhesives which is commonly used in the field of sanitary napkins or panty liners is crimping. However crimping by its very nature reduces material strength such that using it to create a laminate which is mechanically strained afterwards does create severe problems of material stability for the laminate. Therefore crimping and mechanical straining, in particular ring-rolling are not compatible in the same location and have to be kept separate by the space within which they typically fall due to process variations.

It is therefore an objective of the present invention to provide a high speed mechanical straining process for laminates which are used in making disposable absorbent articles comprising zones of extensibility. It is another objective of the present invention to provide an improvement in that the process developed according to the present invention can be carried out at temperatures similar to those of today's adhesive materials, while eliminating the problems associated with the stickiness of adhesives below their application temperature.

These and other objectives of the present invention will become more pronounced from the further description of the invention below.

### Summary of the invention

The present invention provides a high speed mechanical straining process to provide laminates for use in disposable absorbent articles having extensible zones. In particular sanitary napkins having side flaps to automatically wrap the side edges of the undergarment of a wearer of the sanitary napkin are supplied with extensible zones according to the high speed process of the present invention. The process according to the present invention comprises the following steps:
- providing a first and a second continuos layer at least one of which is, preferably both are, strainable and at least one of which is liquid pervious.
- conveying the layers in a machine direction which is the production direction for the laminates and typically for the disposable absorbent articles as well.
- joining the layers including the liquid pervious layer by soldering to provide the laminate. The soldering uses a solder which is non-sticky at 20°C or below, preferably a solder which is non-sticky at 40°C or below. The solder is applied at a temperature above its solidifying temperature to either of the two layers which are contacted thus forming the laminate.
- mechanically straining the laminate to render the designated zones extensible in order to form the extensible zones in the laminates for use in the disposable absorbent articles.

For the preferred sanitary napkins or panty liners made according to the present invention the extensible zones are placed such that they allow the side flaps to conform to the individual shape of the side edges of the crotch portion of undergarments in which the sanitary napkins or panty liners are worn.

The high speed mechanical straining process is operating at a surface speed of the laminate of at least 0.5 m/s and preferably at least 1.5m/s. The straining can be parallel, perpendicular or at an angle in relation to the machine direction in which the laminate is transported during the mechanical straining step. The mechanical straining is preferably provided by ring rolling according to the above identified prior art references.

### Brief description of the drawings

Figure 1 is an exploded view of a sanitary napkin made according to the present invention not showing the solder but identifying the various elements of the preferred sanitary napkins or panty liners as representative examples of disposable absorbent articles having side flaps.
Figure 2 shows a top plan view of a sanitary napkin having ring rolled zones of extensibility in the side flaps. The zones are indicated by hatched lines symbolizing that they are angled to the longitudinal axis.

### Detailed description of the invention

According to the present invention a high speed mechanical straining process to generate extensible zones in laminates for use in the disposable absorbent articles is provided. The laminates comprise at least two layers at least one of which is liquid pervious and at least one is, preferably both are, strainable. The layers are joined at least partially by soldering.

The absorbent article has a body facing surface, typically provided by a liquid impermeable substrate of fibrous or film like structure; a garment facing surface, preferably provided by a liquid impermeable, but breathable substrate and an absorbent structure placed between the body facing surface and the garment facing surface. The absorbent article has a longitudinal axis (10) and a lateral axis (11) as shown in the Figures and can comprise any of the components or features usual in the art including in particular side flap components and any sort of extensibility or elastication feature known in the art.

The preferred sanitary napkin or panty liner made according to the present invention has a pair of side wrapping elements (or "undergarment covering components") that provide coverage to the wearer's panties to reduce side soiling (i.e., staining of the edges of the panty crotch) without the use of conventional flaps.

The preferred sanitary napkin or panty liner comprises a main body portion comprising a liquid pervious topsheet, a liquid impervious backsheet joined to the topsheet, and an absorbent core positioned between the topsheet and the backsheet. The side wrapping elements are either integrally formed with the main body portion which is preferred or joined to the garment-facing side of the main body portion inboard of the longitudinal side edges thereof.

The side wrapping elements are each provided with at least one zone of extensibility, preferably two spaced apart zones of extensibility, which are symmetrically placed in respect to the longitudinal centerline of the absorbent article. The zones of extensibility are regions of the side wrapping elements that have a greater range of extension than the adjacent regions of the side wrapping elements.

The disposable article for absorbent liquid is described below by reference to a sanitary napkin or panty liner. However products such as adult or baby diapers or adult incontinence inserts comprising zones of extensibility can similarly benefit from the process of the present invention.

If the side wrapping elements are formed integrally with the main body portion of the absorbent article, notch regions are formed extending around the intersection between the perimeter of the side wrapping elements and the longitudinal side edge of the main body portion where the side wrapping elements extend beyond the longitudinal side edge of the main body portion. For these integral side wrapping element designs it is preferable when at least one symmetric pair of zones of extensibility extend into said notch regions, i.e. each extensible zone extends across the longitudinal side edge of the main body portion.

Alternatively for side wrapping elements which are provided as separate elements and which are attached to said garment-facing side (and which are unattached outward from where they are attached) the distance between the laterally most inward points of the zones of extensibility and the longitudinal centerline is preferably in the range of 40 mm to 50 mm.

Generally the zone of extensibility can be primarily extensible in the longitudinal direction or primarily in the transverse direction or in any direction falling therebetween. For integrally formed side wrapping elements the zone of extensibility is most preferably extensible in a direction following as close as possible the adjacent outer perimeter of the absorbent article. Preferably the zones of extensibility are provided with corrugations having fold lines.

The sanitary napkins of the present invention provide an alternative to conventional sanitary napkins having side flaps. In one embodiment the side wrapping elements require no action on the part of the wearer to fold them under her panties or to attach them to the panties. The side wrapping elements stay in place well enough to cover the sides edges of the wearer's panties without affixing them underneath the wearer's panties.

In an alternative embodiment, particularly for side wrapping elements extending far outside the main body portion, the sanitary napkin may be provided with a fastener, such as a pressure sensitive adhesive. The adhesive fastener may be provided on the garment-facing side of the main body portion and also extend onto the garment-facing side of the side wrapping elements. In this embodiment, particularly in narrow panty crotches, the side wrapping elements may fold around the side edge of the wearer's panty crotch so that portions of the side wrapping elements even overlap. This forms a novel structure that pinches the side edge of the panties between the folded portion of the side wrapping elements.

### Topsheet

The topsheet (2) is compliant, soft feeling, an non-irritating to the wearer's skin. The topsheet also can have elastic characteristics allowing it to be stretched in one or two directions in portions of the topsheet or throughout its extension. Further, the topsheet is fluid pervious permitting fluids (e.g., menses and/or urine) to readily penetrate through its thickness. A suitable topsheet can be manufactured from a wide range of materials such as woven and non woven materials; polymeric materials such as apertured formed thermoplastic films, apertured plastic films, and hydroformed thermoplastic films; porous foams; reticulated foams; reticulated thermoplastic films; and thermoplastic scrims. Suitable woven and nonwoven materials can be comprised of natural fibers (e.g., wood or cotton fibers), synthetic fibers (e.g., polymeric fibers such as polyester, polypropylene, or polyethylene fibers) or from a combination of natural and synthetic fibers.

Preferred topsheets for use in the present invention are selected from high loft nonwoven topsheets and apertured formed film topsheets. Apertured formed films are especially preferred for the topsheet because they are pervious to body exudates and yet non absorbent and have a reduced tendency to allow fluids to pass back through and rewet the wearer's skin. Thus, the surface of the formed film that is in contact with the body remains dry, thereby reducing body soiling and creating a more comfortable feel for the wearer. Suitable formed films are described in U.S. Patent 3,929,135; U.S. Patent 4,324,246; U.S. Patent 4,342,314; U.S. Patent 4,463,045; and U.S. Patent 5,006,394. Particularly preferred microapetured formed film topsheets are disclosed in U.S. patent 4,609,518 and U.S. patent 4,629,643. A preferred topsheet for the present invention comprises the formed film described in one or more of the above patents and marketed on sanitary napkins by The Procter & Gamble Company of Cincinnati, Ohio as "DRI-WEAVE".

Topsheets having not a homogeneous distribution of liquid passage ways but only a portion of the topsheet comprising liquid passage ways are also contemplated by the present invention. Typically such topsheets would have the liquid passage ways oriented such that they result in a centrally permeable and peripherally impermeable topsheet for liquids.

The body surface of the formed film topsheet can be hydrophilic so as to help liquid to transfer though the topsheet faster than if the body surface was not hydrophilic. In a preferred embodiment, surfactant is incorporated into the polymeric materials of the formed film topsheet such as is described in PCT-publication WO 93/09741. Alternatively, the body surface of the topsheet can be made hydrophilic by treating it with a surfactant such as is described in U.S. 4,950,254.

Another alternative are so called hybrid topsheets which incorporate fibrous and film like structures particularly useful embodiments of such hybrid topsheets are disclosed in PCT publications WO 93/09744; WO 93/11725 or WO 93/11726.

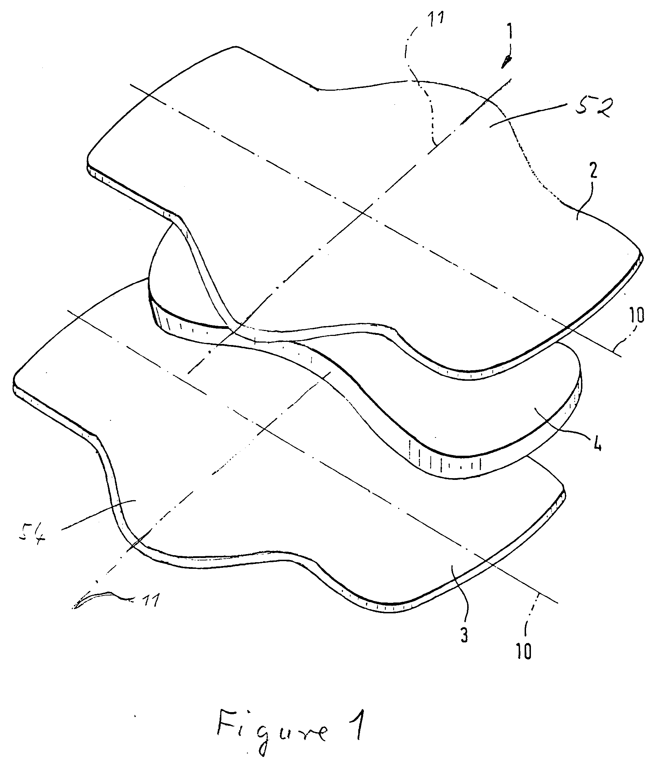

The topsheet (2) typically extends across the whole of the absorbent structure (4) and outside the area coextensive with the absorbent structure (4). As indicated in Figure 1 the topsheet (2) extends and forms part or all of the preferred side flaps as shown and designated 52 in Figure 1.

When referring to the topsheet a multi layer structure or a mono layer structure are contemplated. If the various layers of multilayer topsheets are joined to each other they are as such susceptible to the joining step according to the present invention prior to mechanical straining.

The hybrid topsheet mentioned above is such a multi layer design but other multilayer topsheets such as primary and secondary topsheet designs are also considered susceptible to be joined by the process according to the present invention

### Absorbent structure

The absorbent structure is shown as a single entity (4) in Figure 1. It can include the following components: (a) optionally a primary fluid distribution layer preferably together with a secondary fluid distribution layer; (b) a fluid storage layer; (c) optionally a fibrous ("dusting") layer underlying the storage layer; and (d) other optional components.

### a Primary/Secondary Fluid Distribution Layer

One optional component of the absorbent structure according to the present invention is a primary fluid distribution layer and a secondary fluid distribution layer. The primary distribution layer typically underlies the topsheet and is in fluid communication therewith. The topsheet transfers the acquired fluid to this primary distribution layer for ultimate distribution to the storage layer. This transfer of fluid through the primary distribution layer occurs not only in the thickness, but also along the length and width directions of the absorbent product. The also optional but preferred secondary distribution layer typically underlies the primary distribution layer and is in fluid communication therewith. The purpose of this secondary distribution layer is to readily acquire fluid from the primary distribution layer and transfer it rapidly to the underlying storage layer. This helps the fluid capacity of the underlying storage layer to be fully utilised.

### b Fluid Storage Layer

Positioned in fluid communication with, and typically underlying the primary or secondary distribution layers, is a fluid storage layer. The fluid storage layer can comprise any usual absorbent material or combinations thereof. It preferably comprises absorbent gelling materials usually referred to as "hydrogel", "superabsorbent" "hydrocolloid" materials in combination with suitable carriers.

The absorbent gelling materials are capable of absorbing large quantities of aqueous body fluids, and are further capable of retaining such absorbed fluids under moderate pressures. The absorbent gelling materials can be dispersed homogeneously or non-homogeneously in a suitable carrier. The suitable carriers, provided they are absorbent as such, can also be used alone.

Suitable absorbent gelling materials for use herein will most often comprise a substantially water-insoluble, slightly cross linked, partially neutralised, polymeric gelling material. This material forms a hydrogel upon contact with water. Such polymer materials can be prepared from polymerizable, unsaturated, acid-containing monomers. Suitable unsaturated acidic monomers for use in preparing the polymeric absorbent gelling material used in this invention include those listed in U.S. Patent 4,654,039 reissued as RE 32,649. Preferred monomers include acrylic acid, methacrylic acid, and 2-acrylamido-2-methyl propane sulfonic acid. Acrylic acid itself is especially preferred for preparation of the polymeric gelling material.

Suitable carriers include materials which are conventionally utilised in absorbent structures such as cellulose fibers, in the form of fluff and /or tissues. Suitable carriers can be used together with the absorbent gelling material, however they can also be used alone or in combinations. Most preferred are tissue or tissue laminates in the context of sanitary napkins /party liners.

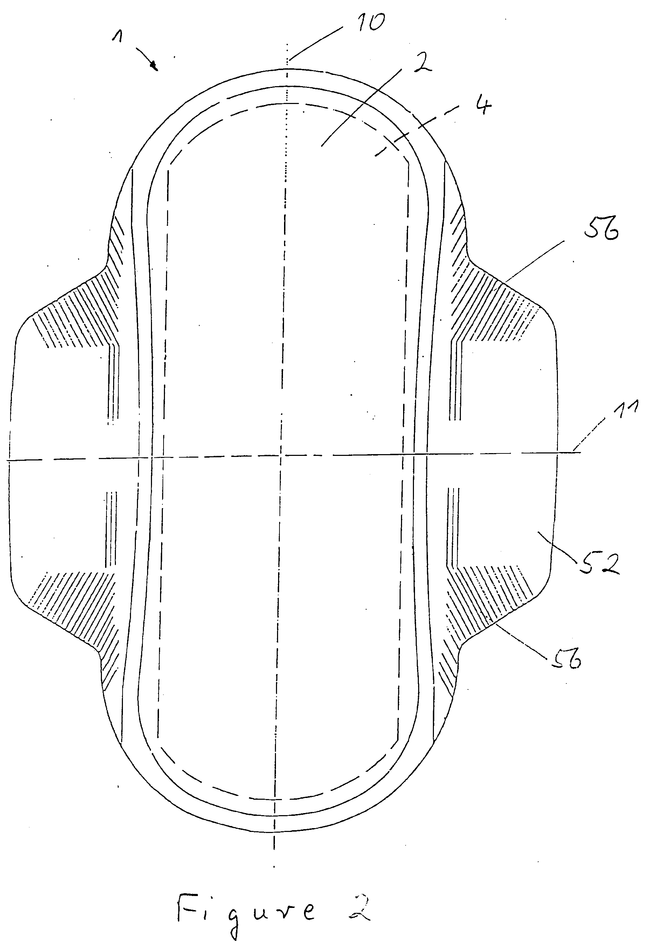

An embodiment made according to the present invention is shown in Figure 2 where the absorbent structure (4) comprises a double layer (42) tissue laminate formed by folding the tissue onto itself. These layers can be joined by soldering both layers to each other.

Modified cellulose fibers such as the stiffened cellulose fibers can also be used. Synthetic fibers can also be used and include those made of cellulose acetate, polyvinyl fluoride, polyvinylidene chloride, acrylics (such as Orlon), polyvinyl acetate, non-soluble polyvinyl alcohol, polyethylene, polypropylene, polyamides (such as nylon), polyesters, bicomponent fibers, tricomponent fibers, mixtures thereof and the like. Preferably, the fiber surfaces are hydrophilic or are treated to be hydrophilic. The storage layer can also include filler materials, such as Perlite, diatomaceous earth, Vermiculite, etc., that lower rewet problems.

If the absorbent gelling material is dispersed non-homogeneously in a carrier, the storage layer can nevertheless be locally homogenous, i.e. have a distribution gradient in one or several directions within the dimensions of the storage layer. Non-homogeneous distribution can also refer to laminates of carriers enclosing absorbent gelling materials partially or fully.

### c Optional Fibrous ("Dusting") Layer

An optional component for inclusion in the absorbent structure according to the present invention is a fibrous layer adjacent to, and typically underlying the storage layer. This underlying fibrous layer is typically referred to as a "dusting" layer since it provides a substrate on which to deposit absorbent gelling material in the storage layer during manufacture of the absorbent structure. Indeed, in those instances where the absorbent gelling material is in the form of macro structures such as fibbers, sheets or strips, this fibrous "dusting" layer need not be included. However, this "dusting" layer provides some additional fluid-handling capabilities such as rapid wicking of fluid along the length of the pad.

### d Other Optional Components of the absorbent structure

The absorbent structure according to the present invention can include other optional components normally present in absorbent webs. For example, a reinforcing scrim can be positioned within the respective layers, or between the respective layers, of the absorbent structure. Such reinforcing scrims should be of such configuration as to not form interfacial barriers to fluid transfer, especially if positioned between the respective layers of the absorbent structure. Given the structural integrity that usually occurs as a result of thermal bonding, reinforcing scrims are usually not required for the absorbent structures according to the present invention.

Another component which can be included in the absorbent structure according to the invention and preferably is provided close to or as part of the primary or secondary fluid distribution layer are odor control agents. Typically active carbon coated with or in addition to other odor control agents, in particular suitable zeolite or clay materials, are optionally incorporated in the absorbent structure. These components can be incorporated in any desired form but often are included as discrete particles.

### Backsheet

The backsheet (3) primarily prevents the exudates absorbed and contained in the absorbent structure from wetting articles that contact the absorbent product such as underpants, pants, pyjamas and undergarments. The backsheet (3) is preferably impervious to liquids (e.g. , menses and/or urine) and is preferably manufactured from a thin plastic film, although other flexible liquid impervious materials can also be used. As used herein, the term "flexible" refers to materials that are compliant and will readily conform to the general shape and contours of the human body. The backsheet also can have elastic characteristics allowing it to stretch in one or two directions.

The backsheet typically extends across the whole of the absorbent structure and can extend into and form part of or all of the preferred sideflaps as shown and designated 54 in Figure 1.

The backsheet can comprise a woven or nonwoven material, polymeric films such as thermoplastic films of polyethylene or polypropylene, or composite materials such as a film-coated nonwoven material. Preferably, the backsheet is a polyethylene film having a thickness of from about 0.012 mm (0.5 mil) to about 0.051 mm (2.0 mils).

Exemplary polyethylene films are manufactured by Clopay Corporation of Cincinnati, Ohio, under the designation P18-0401 and by Ethyl Corporation, Visqueen Division, of Terre Haute, Indiana, under the designation XP-39385. The backsheet is preferably embossed and/or matte finished to provide a more clothlike appearance. Further, the backsheet can permit vapours to escape from the absorbent structure, i.e. be breathable, while still preventing exudates from passing through the backsheet. Also breathable backsheets comprising several layers which are joined to each other can be used and are preferably joined by the method according to the present invention.

Sanitary napkins and panty liners preferably comprise sideflaps also called side wrapping elements. The function of side wrapping elements, whether integral or joined to the article after being formed separately, is further improved by rendering them extensible in one or both directions parallel to the longitudinal axis (10) or lateral axis (11). The extensibility can be provided across all or only part of the side wrapping elements and can be achieved by pleating or ring-rolling those parts which are to be rendered extensible.

If side flaps are formed from laminates of topsheet and backsheet - such as those shown in Figure 1 or 2 - the soldering according to the present invention can be used particularly beneficial. It eliminates the problems associated with built up of tacky adhesive on high speed straining equipment parts such as the rolls used for ring-rolling.

In the preferred sanitary napkin/panty liner made according to the present invention shown in FIG. 2, the side wrapping elements each comprise laminate having two zones of extensibility 56 therein. The extensibility of all the zones of extensibility 56 on the side wrapping elements can be in the same direction. Alternatively, one or more of the zones of extensibility 56 may be extensible in a different direction. Also the extensibility in each zone 56 can vary if so desired.

The zones of extensibility 56 are preferably rendered capable of extending between about 20% and about 90%, more preferably between about 50% and about 80%, and most preferably between about 70% and about 80% under the forces associated with wearing the sanitary napkin in a pair of panties. The zones of extensibility 56 are also preferably extensible without being elastic. Further, any inherent elasticity in the zones of extensibility 56 (that is, any tendency of the material comprising the zones of extensibility to return to its original dimension) is generally relatively low to non-existent.

### Process steps according to the present invention

The first process step is to provide at least two layers for being joined to form a laminate in accordance with the present invention. Usually the layers are provided at a fairly high surface speed as roll goods. They can form separate layers to be combined with other materials or other structural entities thereafter. Preferably they form structural entities integral with the absorbent article, particularly they are the topsheet and backsheet forming integral side wrapping elements of sanitary napkins or panty liners.

The surface speed at which the joining process needs to take place is at least 0.5 m/s and more preferably at least 1.5 m/s. The movement is typically in the longitudinal direction (10) of the absorbent articles which therefore is parallel to the machine direction of the absorbent articles made according to the present invention. Movements parallel to the transverse axis (11) are also possible but less often used.

The next step according to the present invention is the joining of the layers provided and conveyed according to the present invention by soldering them to each other. The soldering is considered useful only to provide permanent connections between materials since the formed structures typically have to be coherent.

The soldering should withstand at least a peel strength of 0.4 N/2,5 cm. The peel strength is the strength required to peel apart the materials joined to each other on a sample strip of 2.5 cm width. A full test description is included in the following. The soldering strength can of cause not exceed the material strengths of the materials joined. Therefore an alternative to the test against 0.4 N/2.5 cm can be the internal cohesion test. In this test a soldered connection is delaminated. The soldered connection satisfies the test if one of the materials is destroyed.

Joining materials by soldering requires to apply a solder to one or both surfaces to be joined and bringing the surfaces into contact before the solder cools below its solidifying temperature. In order to apply the solder it is heated to a temperature above its solidifying temperature and applied in a similar fashion or the same fashion as adhesives are applied. As a matter of fact the same equipment used today for applying adhesives can be adapted to apply the solder to the respective surface where it is needed. Methods like contact coating, roll coating, coating by spraying in random or designed patterns (such as swirl coating,) can all be used to apply the solder according to the present invention.

When bringing the surfaces to be joined together the solder contacts both surfaces intimately at a temperature above its solidifying temperature. It creates a permanent connection after cooling below the solidifying temperature. Incorporated within the word soldering according to the present invention are methods which in the metal art are referred to as brazing where the solder forms intermaterial bonds across the contact surface. While not wishing to be limited by theory it is believed that the solder forms a thin layer of intermaterial connections where the top molecular layers of the materials to be soldered to each other are involved.
In order to distinguish the solder from adhesives a stickiness test for defining a solder is described below. Adhesives can be distinguished from the solder according to this test thereby allowing to better understand and repeat the present invention.

Solder materials can be e.g. adhesive materials without the tackifier additives usually used. Alternatively wax type materials of high coherent strength with melting points similar to those of hot melt adhesives can be employed. One such solder example is a cleaning material designated Fuller HS350. New, available from the H.B. Fuller Company, Lueneburg, Germany. The operating temperature of this solder is about 120°C to 140°C while comparable hot melt adhesives such as Findley 990-374-b, available from Findley, Rosendaal, The Netherlands, have operating temperatures of about 135° to 145°C.

The last necessary step in order to provide the laminates having extensible zones is the mechanical straining in order to render the zones extensible designated to become extensible. As discussed above mechanical straining can be provided by any of the methods known in the art. However the most preferred method is ring rolling according to the above identified prior art publications. In the ring rolling step two rolls of meshed grooves interlock and thereby force material transported through the rolls to extend perpendicular to the groove direction. Since one of the materials forming the laminate is liquid permeable the solder in these high speed processes will penetrate the apertures providing the permeability and would typically cause build up on the ring rolling process. This is prevented by use of a solder having no stickiness at the point of mechanical straining any more.

It therefore has finally become possible to manufacture extensible zones in laminates which laminates are permanently joined prior to the mechanical straining used for rendering designated zones of the laminate extensible.

Not all material layers undergoing mechanical straining need to be strainable. By "strainable" it is meant that the material undergoes permanent deformation without destruction of its integrity. However the combination of non-strainable with strainable material can be strained. For example non-strainable non-woven with strainable polymeric film could be strained. In this case the film is permanently deformed (and provides the desired zone of extensibility) while the non-woven would be locally destroyed (fibres or fibre bonds breaking) but otherwise remain firmly soldered to the film.

### Test methods:

All tests conducted in respect to the present invention require test conditions of 21° plus minus 1° C and relative humidity of 50% unless stated otherwise. All test materials are conditioned at this temperature and humidity for at least 4 hours prior to the test itself.

### Peel strength or peel force testing

The peel strength test analyses the force required to delaminate a connection between materials when one material is peeled from the other material at a 180 degree angle. In respect to defining a permanent connection it has been found most sensible to create a realistic sample of the soldered material connection and analyse the peel strength between the actual materials to be soldered to each other rather than attempt to standardise the materials or solder application. The test is conducted on a sample strip of 2.5 cm width having sufficient end tabs to apply the peel force equally across the whole width of the sample to be tested.

The peel strength of a soldered connection between materials is sufficient if the force required to delaminate the soldered connection is 0.4 N/2.5 cm or if the connection can withstand holding a load of 40g without delaminating.

As already indicated materials having a cohesive strength less than the required 0.4 N/2.5 cm are still considered permanently connected by soldering if the material experiences destructive failure rather than the soldered connection. Obviously this test is substantially easier in executing than the force measurement of the peel strength. It can also be used on materials having higher cohesive strength than 0.4 N/2.5 cm provided one of the materials and not the soldered connection is destroyed. Therefore in general destruction of one of the materials soldered together implies that the soldered connection was permanent.

### The stickiness test

The stickiness test attempts to distinguish a solder material from a tacky adhesive. Typically the distinction will be quite apparent when simply touching an open surface of a solder material versus an open surface of an adhesive. However in order to define the distinction the following test methodology has to be applied.

### Principal stickiness test method steps

- melt the test solder material to be analysed for stickiness according to the supplier instruction;
- apply the molten test solder material to a substrate;
- let the molten test solder material and substrate cool to the test temperature while ensuring that the open side of the test material remains untouched and is only exposed to clean air;
- apply a second substrate, the stickiness substrate, to the untouched side of the test material under a certain pressure;
- measure the force required to peel the second substrate from the first substrate.

A solder material will have a peel force of zero at the test temperature while test materials having a peel force greater than zero are not useful in the process according to the present invention.

The test material should be heated/molten to the temperature according to the manufacturers instruction of the test material. Care should be taken with test solders which undergo curing after being heated and cooled down. Even so they may appear sticky prior to heating they can be non-sticky after being applied; i.e. after curing.

The test solder material to be checked is applied to a substrate in the usual way in which adhesives would be applied. For the current test method it has been found that using a Nordson slot coater with a 50mm wide nozzle available from a Nordson, Lueneburg, Germany is useful. The solder is applied for this test in a width of at least 2.5 cm full surface coating at a speed of the first substrate of at least 0.5 m/s relative to the coating equipment. Wider coatings are acceptable while coatings of smaller width or only fractions of the width being coated (e.g. by spiral coating) are non-acceptable in the context of this test. Of course such coating patterns can be used for applying the solder in the particular application context according to the process of the present invention.

The length of the solder material applied should be sufficiently long to conduct a series of tests in order to provide at least ten samples. The individual test sample length should be no less than 10 cm. The amount of solder used according to the stickiness test method should be the same as intended to be used in the desired process according to the present invention. A minimum quantity is however such that the test surface onto which the solder is applied is fully covered by the test solder, and amounts of 10 to 30 g/m² have been found useful.

The first substrate onto which the solder is applied according to the test should be a polyethylene film having a thickness of between 20 and 30 micrometers. Solders having application temperatures too high to be applied to polyethylene films will usually not be considered at all in the context of absorbent articles. Should however in a specific case a solder be considered having an application temperature so high that the polyethylene film would not be able to withstand the application of the test solder (e.g. burn through) then a polypropylene film, or in extreme cases even a polyethylene-terephthalate film can be substituted. It should however be considered that solders having a too high application temperature will not be useful in the context of disposable absorbent articles and typical application temperatures are well below 200°C, usually below 150°C; except for soldering tissue materials useful in manufacturing disposable absorbent articles.

After the solder has been applied to the substrate it is allowed to cool to the temperature at which measurements are to be taken. It has been found that one critical temperature is about 20°C since most manufacturing processes run around room temperature, i.e. about 20°C. The other typically critical temperature is the body temperature of the wearer of disposable absorbent articles since according to the objectives of the present invention any stickiness of the solder to the skin of the wearer should be prevented. Including a safety margin 40°C is considered to be an adequate upper limit below which the solder material has to be non-sticky according to this stickiness test to achieve this objective. Therefore two qualities of solder can be distinguished one which is non-sticky at 20°C or below and one preferred quality which is non-sticky at 40°C or below.

The next step according to the test method is the application of a second substrate. The second substrate should extend to the full surface of the solder test samples and preferably exceed the test surface sufficiently to provide edges where it can be manipulated.

Ideally the second substrate should provide the surface onto which the solder should display its lack of stickiness. However for test standardisation two materials are considered critical.

One substrate material is a woven cotton surface. It can be obtained under the designation "white, 100% cotton weave, style # 429-W, available from Loeffler Sitter Technic GmbH, Nettersheim, Germany. The second alternative substrate material is a polyethylene film of 25 micrometer thickness available under the designation "Tacolin Polyethylene Film Code ST 400" available from Taco Plastics, Manchester, Great Britain.

Ten samples each are tested by applying the second substrate to the open surface of the solder under a pressure of 0.4 N/cm. The application of the pressure can be conducted by using a weight in accordance with the size of the test surface area (for the minimum test surface area this weight would be 1 kg) and extending essentially to the test surface.

Upon application of the test weight care should be taken that no uneven weight distribution is introduced e.g. by tilting the weight. For the preferred solders according to the present invention the test result will not alter over a wide range of compressions of up to 4 N/cm² or even 10 N/cm².

The compression under which the second substrate is applied to the solder surface should be maintained for at least 30 seconds up to 1 minute. The weight is then removed and the samples are left for another 30 seconds to 1 minute.

The measurement of peel strength then can be conducted by attempting to peel one surface away from the other and measuring the required force with highly sensitive force gauges. This measurement is well known in the art to distinguish between the strength of different adhesives. Due to the attempt to measure a zero force value (i.e. non-stickiness) the force measurement creates substantial problems in respect to the accuracy of the results. When measuring an absolute value of zero the variation, also known as measurement noise, is strongly amplified. Therefore it has been found that the stickiness test identifying the solder is satisfied if the second substrate delaminates under its own gravitational force.

This is measured by taking the sample, preferably while touching only the outer edges of the first substrate, and turning the sample such that the second substrate is held under its own gravitational force only by the stickiness of the test solder. If the second substrate delaminates and falls off within less than 5 seconds, preferably right away, the test solder satisfies the criterium of non-stickiness.

Samples where the second substrate remains joined to the first substrate are not considered solder materials in the context of the present invention. For these samples a value of stickiness can be measured as indicated above. Of course great care must be taken to ensure that no other forces (e.g. electrostatic forces) are involved in particular if the test is conducted with polyethylene films as the two substrates.

### Selective solder materials

Selective solder materials which are non-sticky in conjunction with particular surfaces but sticky on "other" surfaces are also contemplated according to the process of the present invention. Their use is of course limited to joining materials where the surfaces which may get into contact with the solder (such as the steel surfaces of machinery or human skin) are not any of the mentioned "other" surfaces.

For example the absorbent structure (4) shown in Figure 2 as having two layers (42) may employ a selective solder. The solder in this context could be e.g. sticky to human skin even after cooling down to room temperature since the absorbent structure 4 will be fully enclosed in the backsheet (3) and the top sheet (2).

One material a solder can be sticky to is another layer of the same solder. This characteristic is in line with the present invention since a layer of solder could be applied to each of the two surfaces to be joined. A certain stickiness of solder to solder even below the solidifying temperature would then usually be welcome since it improves the permanent connection formed according to the present invention.

## Claims

1. High speed process to manufacture disposable absorbent articles (1) having extensible zones (56) said zones (56) being provided by laminates said process comprising the steps of
- providing a first and a second continuous layer (2,3), at least one of said layers being strainable and at least said first layer (2) being liquid pervious;
- conveying said first and said second layer (2,3) in a machine direction at a surface speed of at least 0,5 m/s;
- providing a solder which is heated to a temperature above its solidifying temperature, said solder being non-sticky at 20°C or below;
- continuously joining said first and said second layer (2,3) by depositing said solder onto one of said first or said second layer (23) before said solder cools below its solidifying temperature and contacting both layers to provide a laminate,
- mechanically straining said laminate to render designated zones (56) of said laminate extensible.

2. Process according to claim 1 wherein said disposable absorbent articles (1) are sanitary napkins or panty liners used in the crotch portion of an undergarment, said articles (1) have side flaps (52,54), said side flaps (52,54) comprising said laminate and extending beyond and wrapping around the side edge of the crotch portion of said undergarment, said extensible zones (56) in said laminate being located so as to allow said side flaps (52,54) to conform to the individual shape of said side edges of said crotch portion of said undergarment.

3. Process according to any of the preceding claims wherein said laminate is conveyed in a machine direction at a surface speed of at least 1.5m/s when starting said straining step.

4. Process according to any of the preceding claims wherein said straining is conducted in a direction parallel to said machine direction.

5. Process according to any of the claims 1 to 2 wherein said straining is conducted in a direction perpendicular to said machine direction.

6. Process according to any of the claims 1 to 3 wherein said straining is conducted at an angle to said machine direction.

7. Process according to any of the preceding claims wherein said soldering uses a solder which is non-sticky at 40°C or below.

8. Process according to any of the preceding claims wherein said straining step uses a ring-rolling process.

## Patentansprüche

1. Hochgeschwindigkeitsverfahren zur Herstellung wegwerfbarer absorbierender Artikel (1), welche verlängerbare Zonen (56) aufweisen, wobei die genannten Zonen (56) durch Laminate beigestellt sind, wobei das genannte Verfahren folgende Schritte umfaßt
- Beistellen einer ersten und einer zweiten kontinuierlichen Schichte (2, 3), wobei mindestens eine der genannten Schichten dehnbar ist und mindestens die genannte erste Schichte (2) flüssigkeitsdurchlässig ist;
- Befördern der genannten ersten und der genannten zweiten Schichte (2, 3) in einer Maschinenrichtung bei einer Oberflächengeschwindigkeit von mindestens 0,5 m/s;
- Beistellen eines Lötmittels, welches auf eine Temperatur oberhalb seiner Verfestigungstemperatur erhitzt wird, wobei das genannte Lötmittel bei 20°C oder darunter nicht-klebrig ist;
- kontinuierliches Verbinden der genannten ersten und der genannten zweiten Schichte (2, 3) durch Ablegen des genannten Lötmittels auf eine der genannten ersten oder der genannten zweiten Schichte (2, 3) und Berühren beider Schichten, bevor das genannte Lötmittel unterhalb seiner Verfestigungstemperatur abkühlt, um ein Laminat beizustellen,
- mechanisches Recken des genannten Laminats, um bezeichnete Zonen (56) des genannten Laminats verlängerbar zu machen.

2. Verfahren nach Anspruch 1, bei welchem die genannten wegwerfbaren absorbierenden Artikel (1) Hygienevorlagen oder Slipeinlagen sind, welche im Schrittabschnitt eines Unterwäschestücks verwendet werden, wobei die genannten Artikel (1) Seitenlappen (52, 54) aufweisen, die genannten Seitenlappen (52, 54) das genannte Laminat umfassen und sich über den Seitenrand des Schrittabschnitts der genannten Unterwäsche hinaus erstrecken und diesen umhüllen, wobei die genannten verlängerbaren Zonen (56) im genannten Laminat so angeordnet sind, um den genannten Seitenlappen (52, 54) zu gestatten, sich an die einzelne Gestalt der genannten Seitenränder des genannten Schrittabschnitts des genannten Unterwäschestücks anzupassen.

3. Verfahren nach einem der vorhergehenden Ansprüche, bei welchem das genannte Laminat in einer Maschinenrichtung bei einer Oberflächengeschwindigkeit von mindestens 1,5 m/s transportiert wird, wenn der genannte Schritt des Reckens begonnen wird.

4. Verfahren nach einem der vorhergehenden Ansprüche, bei welchem das genannte Recken in einer Richtung parallel zur genannten Maschinenrichtung ausgeführt wird.

5. Verfahren nach einem der Ansprüche 1 bis 2, bei welchem das genannte Recken in einer Richtung lotrecht zur genannten Maschinenrichtung ausgeführt wird.

6. Verfahren nach einem der Ansprüche 1 bis 3, bei welchem das genannte Recken in einem Winkel zur genannten Maschinenrichtung ausgeführt wird.

7. Verfahren nach einem der vorhergehenden Ansprüche, bei welchem das genannte Löten ein Lötmittel verwendet, welches bei 40° C oder darunter nicht-klebrig ist.

8. Verfahren nach einem der vorhergehenden Ansprüche, bei welchem der genannte Schritt des Reckens ein Ringwalzverfahren verwendet.

## Revendications

1. Procédé à grande vitesse pour fabriquer des articles absorbants jetables (1) ayant des zones extensibles (56), lesdites zones (56) étant fournies par des stratifiés, ledit procédé comprenant les étapes consistant à :
- fournir une première couche continue et une deuxième couche continue (2, 3), au moins une desdites couches étant déformable et au moins ladite première couche (2) étant perméable aux liquides ;
- transporter ladite première couche et ladite deuxième couche (2, 3) dans le sens machine à une vitesse de surface d'au moins 0,5 m/s ;
- fournir une soudure qui est chauffée jusqu'à une température supérieure à sa température de solidification, ladite soudure étant non adhérente à 20°C ou à une température inférieure ;
- réunir de manière continue ladite première couche et ladite deuxième couche (2, 3) en déposant ladite soudure, sur l'une de ladite première couche, ou de ladite deuxième couche (23) avant que ladite soudure refroidisse en dessous de sa température de solidification, et en mettant en contact les deux couches afin de réaliser un stratifié ;
- déformer mécaniquement ledit stratifié afin de rendre extensibles des zones désignées (56) dudit stratifié.

2. Procédé selon la revendication 1, dans lequel lesdits articles absorbants jetables (1) sont des serviettes hygiéniques ou des garnitures de culotte utilisées dans la partie d'entrejambe d'un sous-vêtement, lesdits articles (1) ont des rabats latéraux (52, 54), lesdits rabats latéraux (52, 54) comprenant ledit stratifié et s'étendant au-delà du bord latéral de la partie d'entrejambe dudit sous-vêtement, et s'enroulant autour de ce bord latéral, lesdites zones extensibles (56) dudit stratifié étant situées de manière à permettre que lesdits rabats latéraux (52, 54) épousent la forme individuelle desdits bords latéraux de ladite partie d'entrejambe dudit sous-vêtement.

3. Procédé selon l'une quelconque des revendications précédentes, dans lequel ledit stratifié est transporté dans le sens machine à une vitesse de surface d'au moins 1,5 m/s lorsque débute l'étape de déformation.

4. Procédé selon l'une quelconque des revendications précédentes, dans lequel ladite déformation est dirigée dans une direction parallèle audit sens machine.

5. Procédé selon l'une quelconque des revendications 1 et 2, dans lequel ladite déformation est dirigée dans une direction perpendiculaire audit sens machine.

6. Procédé selon l'une quelconque des revendications 1 à 3, dans lequel ladite déformation est dirigée selon un certain angle par rapport au sens machine.

7. Procédé selon l'une quelconque des revendications précédentes, dans lequel ledit soudage utilise une soudure qui est non adhérente à 40°C ou à une température inférieure.

8. Procédé selon l'une quelconque des revendications précédentes, dans lequel ladite étape de déformation utilise un procédé de roulage annulaire.
